# EUROPEAN PATENT SPECIFICATION

(11) **EP 3 199 166 B1**
(45) Date of publication and mention of the grant of the patent: **11.09.2019**
(21) Application number: 15844367.1
(22) Date of filing: 24.09.2015
(51) Int. Cl.: A61K 31/724, A61K 31/7004, A61K 45/00, A61P 35/00

(54) **ANTITUMOR AGENT INCLUDING BETA-CYCLODEXTRIN**
ANTITUMORMITTEL MIT BETA-CYCLODEXTRIN
AGENT ANTITUMORAL COMPRENANT DE LA BETA-CYCLODEXTRINE

(30) Priority: 25.09.2014 US 201462054993 P; 22.01.2015 US 201562106236 P
(43) Date of publication of application: 02.08.2017
(73) Proprietor: JRC G.K., Osaka-shi, Osaka 5500013 (JP)
(72) Inventor: PERKINS, Guy, La Jolla, California 92093 (US); YAMAGUCHI, Ryuji, Osaka-shi Osaka 5500013 (JP)
(74) Representative: Mewburn Ellis LLP
(86) International application number: PCT/JP2015/076956
(87) International publication number: WO 2016/047697

(56) References cited:
- WO-A1-2014/087778
- JP-A- H08 511 041
- ZHANG DONGSHENG ET AL: "2-Deoxy-D-glucose targeting of glucose metabolism in cancer cells as a potential therapy", CANCER LETTERS, NEW YORK, NY, US, vol. 355, no. 2, 10 September 2014 (2014-09-10), pages 176-183, XP029081963, ISSN: 0304-3835, DOI: 10.1016/J.CANLET.2014.09.003
- GROSSE PY. ET AL.: 'Antiproliferative effect of methyl-beta-cyclodextrin in vitro and in human tumour xenografted athymic nude mice' BR. J. CANCER vol. 78, no. ISSUE, 1998, pages 1165 - 1169, XP055420538
- ZHAO X. ET AL.: 'Caveolin-1 negatively regulates TRAIL-induced apoptosis in human hepatocarcinoma cells' BIOCHEMICAL AND BIOPHYSICAL RESEARCH COMMUNICATIONS vol. 378, no. ISSUE, 2009, pages 21 - 26, XP025741107
- LIN T. ET AL.: '2-Tellurium-bridged beta- cyclodextrin, a thioredoxin reductase inhibitor, sensitizes human breast cancer cells to TRAIL- induced apoptosis through DR5 induction and NF- KB suppression' CARCINOGENESIS vol. 32, no. 2, 2010, pages 154 - 167, XP055110705
- KANETO UEKAMA ET AL.: 'Kakushu Methylated Cyclodextrin no Seizai eno Yuko Riyo' THE SYMPOSIUM ON PARTICULATE PREPARATIONS AND DESIGNS KOEN YOSHISHU vol. 23, 2006, pages 1 - 6, XP009501257
- YAMAGUCHI R. ET AL.: 'Efficient Elimination of Cancer Cells by Deoxyglucose-ABT-263/737 Combination Therapy' PLOS ONE vol. 6, no. ISSUE, 2011, page E24102, XP055420546
- MASAHIKO KIKUCHI ET AL.: 'Enhancement of antitumor activity of carmofur (HCFU) by dimethyl-.BETA.-cyclodextrin complexation in P-388 leukemia-bering mice' XENOBIOTIC METABOLISM AND DISPOSITION vol. 3, no. 3, 1988, pages 267 - 273, XP003032474

## Description

### TECHNICAL FIELD

The present invention mainly relates to an antitumor drug comprising β-cyclodextrin (bCD) or a derivative thereof (hereinafter, it may be referred to as simply "β-cyclodextrin" or "bCD", including its derivative) characterized by the combination use with another antitumor drug, an antitumor drug comprising the combination, a combination therapy with bCD and another antitumor drug for treating cancer or the like, etc.

### BACKGROUND ART

A single cancer cell left behind after surgery and/or chemotherapy could cause the recurrence of cancer. Therefore the aim of cancer chemotherapy must be to eliminate all cancer cells. Given the heterogeneity of cancer cells in a tumor, it is difficult to eliminate all cancer cells by a single agent targeting a particular gene product.

We developed a combination therapy of ABT-263 (navitoclax, hereinafter may be referred to as simply "ABT") that can induce apoptosis by inactivating anti-apoptosis protein such as Bcl-2 and Bcl-xL, and 2-deoxyglucose (2DG) that can inhibit glycolysis in a cancer cell, so-called "2-deoxyglucose-ABT-263 (2DG-ABT) combination therapy", and found that the combination of the both drugs can synergistically induce apoptosis (Non-patent Reference 1).

However, the efficiency of the 2DG-ABT combination therapy varied from cell line to cell line. One reason for the varied efficacy is thought to be the varied strength of the phosphoinositide 3-kinase-AKT (PI3K-AKT) pro-survival signal present in cancer cells. Since the PI3K-AKT pathway exists in many normal tissues, targeting this pathway for treating cancer by apoptosis induction can cause many adverse side effects.

On the other hand, receptor tyrosine kinases (RTKs) such as epidermal growth factor receptor (EGFR) and insulin-like growth factor 1 receptor (IGF1R) are also often activated in specific cancer types, leading to the activation of PI3K-AKT, and thus targeting a specific RTK with a specific inhibitor against such receptor or enzyme activating PI3K-AKT may also be an effective way to diminish the pro-survival signal in some cancer cells with fewer side effects. In fact, some drugs for treating cancer which target these receptors have been already developed, and some of them have been actually used in medical practice. However, a real tumor mass is not like cancer cells from one cell line; some cells in a tumor could express IGF1R, while other cells in the same tumor could express EGFR or an insulin receptor. Thus, it is difficult to settle the target cells. In addition, all the receptors can generate a PI3K-AKT pro-survival signal, thus, for example, inhibiting just IGF1R or EGFR or even both would not be enough.

As mentioned above, some drugs inhibiting the signaling between PI3K and AKT which can induce apoptosis as a useful process for treating cancer had been already known, but the effect was not so enough and there were problems such as adverse side effects.

β-Cyclodextrin (bCD) has a conical molecular structure composed of 7 linking sugar chains, which has a unique configuration having a cavity inside. In addition, bCD has both hydrophilic hydroxy groups outside and hydrophobic groups inside, thereby bCD is applied in chemical synthetic field as phase-transfer catalysts, or in biological field using a property of holding another molecular inside (Non-patent Reference 2).

In addition, it is well known that bCD has a property of holding cholesterol inside (Non-patent Reference 3). In plasma membrane, cholesterol has an important bioactivity of transmitting many signals from extracellular to intracellular, and it has been well reported since around 1980 that the group of patients who have a high blood level of cholesterol indicates low cancer risk (Non-patent Reference 4). Thus, there had been no trial to apply bCD targeting cholesterol to cancer therapy, or little trials if any. WO2014/087778 discloses use of hydroxyalkylated β-cyclodextrin as anticancer agent.

### PRIOR ART

### (Non-patent Reference)

[Non-patent Reference 1] Yamaguchi R, at al., PloS one 2011, 6 (9): e24102.
[Non-patent Reference 2] Curr. Top Med. Chem. 2014 14 (3), 330-339
[Non-patent Reference 3] Journal of Lipid Research Vol. 38, 1997, 2264
[Non-patent Reference 4] QJM 2012, 105: 383-388

### SUMMARY OF INVENTION

### (Problems to Be Solved by the Invention)

The purpose of the present invention is mainly to find a drug to induce apoptosis by effectively inhibiting the signaling between PI3K and AKT, which is a useful process to treat cancer or the like.

### (Means to Solve the Problems)

The present inventors have intensively studied and then found that β-cyclodextrin (bCD) which had not been used in treating cancer or the like because bCD has a property of holding cholesterol inside, can unexpectedly attenuate the PI3K-AKT pro-survival signal, induce apoptosis, and then exhibit antitumor activity.

In addition, the present inventors have also found that when bCD is used in combination with 2-deoxyglucose (2DG), 2DG can release a pro-apoptotic protein, Bak from an anti-apoptotic protein, Mcl-1, while bCD inactivates AKT; and when further also used in combination with Bcl-2 antagonist such as ABT-263, Bak can be also released from another anti-apoptotic protein, Bcl-xL, *i.e*., Bak can be completely released from the both proteins Mcl-1 and Bcl-xL to undergo apoptosis. Furthermore, the present inventors have also found that when bCD-2DG is used in combination with an apoptosis inducer other than Bcl-2 antagonists, such as a TNF-related apoptosis-inducing ligand (TRAIL), effective apoptosis can be also undergone.

As mentioned above, the present inventors have found that when administering various antitumor agents in combination with β-cyclodextrin, the effect of the antitumor agents can be enhanced. Based upon the new findings, the present invention has been completed.

The present invention is defined in the claims.

In the present invention, it has been found that targeting cholesterol along with bCD can inhibit the signaling between PI3K and AKT to attenuate AKT pro-survival signals. And it was demonstrated *in vitro* and *in vivo* that, after the signals are attenuated, 2-deoxyglucose (2DG) or other antitumor agents having apoptosis-inducing activity can promote the release of a pro-apoptotic Bak in mitochondria, said release can further promote the release of cytochrome c from mitochondria to induce apoptosis.

### (Effect of the Invention)

In general, it is preferable that antitumor agents are localized only in tumor cells such as cancer cells not to be delivered to the other healthy cells from the viewpoint of side effects. For antitumor agents whose mechanism is apoptosis induction, however, the target site thereof is mitochondria in all cells of the body. Thus, it is difficult to localize such antitumor agents into mitochondria in only tumor cells, and thus it is very difficult to clinically use such antitumor agents having too strong activity, considering the adverse effect to healthy cells.

On the other hand, the present invention is a combination therapy of the plural agents, each of which has a moderate activity as a single agent not to seriously affect healthy cells, but the present invention has a property that the activity can be synergistically enhanced when the plural agents exert each activity in an identical cell.

2DG can act as a mimic of glucose, thus 2DG has the property of penetrating into only cells which have high glucose metabolism through glucose transporters. Such cells having high glucose metabolism are cells in inflamed tissues, over-exercised muscle cells including myocardium, tumor cells such as cancer cells, and brain cells. If suppressing patient's inflammation and controlling patient's over-exercise before treating tumor by the combination therapy of the present invention, 2DG can be almost localized in tumor cells and brain cells.

On the other hand, bCD cannot get through blood-brain barrier, thus bCD exists in body tissues other than brain. Thus, it is possible to almost limit the cells in which the both agents simultaneously exist to only tumor cells, hence the combination therapy of bCD and 2DG can be expected to induce effective apoptosis in tumor cells, which little affect healthy cells.

In addition, the single therapy with bCD or the combination therapy with bCD and an antitumor agent other than 2DG, can completely avoid any adverse effect in brain cells because bCD cannot penetrate into brain cells.

The signal transmission between PI3K and AKT is inhibited with bCD, but the inhibition remains for only few hours, and then the signal transmission recovers in a short time. Using this time-limited activity of bCD, the combination of bCD and the other antitumor agent can induce a strong apoptosis only when the both agents exist in the same cell even if the other antitumor agent is a comparatively lower-potency agent having low side effects. And, the side effect of the combination can be suppressed after the time-limited inhibiting-effect of bCD disappears. Thus, the present combination therapy can achieve an efficient treatment regimen through the administration-timing of each agent in the combination, while the adverse effect for the PI3K-AKT pathway in normal cells can be minimized to reduce the side effect.

### Brief Description of Drawings

Fig. 1 shows the result of Example 1, in which A to C show each result of Tests A to C, respectively.
Fig. 2 shows the results of Examples 2 to 5, in which A shows the result of Example 2, B and C show the result of Example 3, D shows the result of Example 4, and E shows the result of Example 5.
Fig. 3 shows an experimental protocol where the three drugs are administered in the examples.

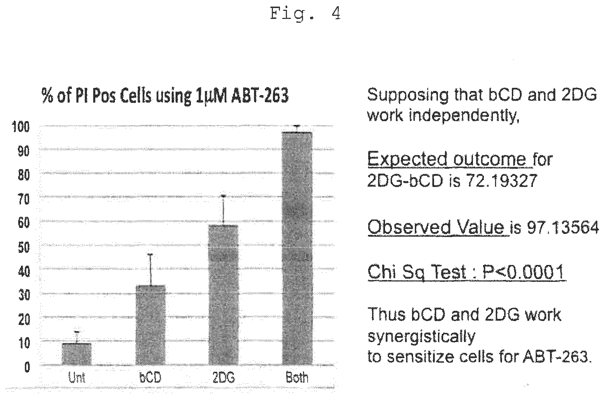

Fig. 4 shows a graph prepared by summarizing the result of 1 µM ABT concentration in Fig. 2, E of Example 5, and calculated values derived from the graphed result.
Fig. 5 shows the result of Example 6.
Fig. 6 shows the result of Example 7.
Fig. 7 shows the result of Example 8.
Fig. 8 shows the result of Example 9.
Fig. 9 shows the result of Example 10.

### Description of Embodiments

β-Cyclodextrin (bCD) has a conical molecular structure composed of 7 linking sugar chains. In the present invention, β-cyclodextrin means β-cyclodextrin itself as well as its derivatives. The derivatives herein mean β-cyclodextrins having various substituents, including methyl-β-cyclodextrin (MBCD), (2-hydroxypropyl)-β-cyclodextrin (HPBCD), carboxymethyl-β-cyclodextrin, carboxymethyl-ethyl-β-cyclodextrin, diethyl-β-cyclodextrin, dimethyl-β-cyclodextrin, glucosyl-β-cyclodextrin, hydroxybutenyl-β-cyclodextrin, hydroxyethyl-β-cyclodextrin, maltosyl-β-cyclodextrin, random methyl-β-cyclodextrin, sulfobutylether-β-cyclodextrin, 2-selenium-bridged β-cyclodextrin, and 2-tellurium-bridged β-cyclodextrin. Preferred β-cyclodextrins include methyl-β-cyclodextrin (MBCD), (2-hydroxypropyl)-β-cyclodextrin (HPBCD), hydroxybutenyl-β-cyclodextrin, hydroxyethyl-β-cyclodextrin, random methyl-β-cyclodextrin, and sulfobutylether-β-cyclodextrin; more preferably, β-cyclodextrin, methyl-β-cyclodextrin (MBCD), and (2-hydroxypropyl)-β-cyclodextrin (HPBCD). Besides bCD, 2-hydroxypropyl-γ-cyclodextrin (HPGCD) which has a good property of holding cholesterol, can be used in the present invention.

bCD or its derivatives used herein can be administered orally or parenterally such as by injection and intravenously.

The dose of bCD is not limited as long as it can inhibit the signal transmission between PI3K and AKT, not seriously affecting patients. For example, bCD can be administered in a dose of 2 to 5000 mg, preferably 2 to 100 mg, per treatment.

The antitumor agent of the present invention that is used in combination with bCD includes 2DG as well as an antitumor agent having apoptosis-inducing action, for example, an agent that can release Bak from Mcl-1 and/or Bcl-xL which are anti-apoptosis proteins, specifically, A-385358, ABT-199, ABT-263 (Navitoclax), ABT-737, AT-101, GX15-070 (obatoclax), HA14-1, oblimersen, and the like, but not limited thereto. Besides, a Fas-related apoptosis-inducing ligand, a TNF-related apoptosis-inducing ligand (TRAIL) and the like can be also used herein, which include, for example, a TRAIL and a derivative thereof (e.g. AMG951), or an antibody that can activate a TRAIL receptor (e.g. mapatumumab, lexatumumab).

In addition, the antitumor agent used herein includes an agent that can induce apoptosis through a signal arising between endoplasmic reticulum and mitochondria, in combination with 2DG-bCD, for example, an inhibitor for HSP90 inhibitors such as gamitrinibs, PU24FC1, PU-H58, PU-H71, and shepherdin; endoplasmic reticulum stress agents; thapsigargin and a derivative thereof such as G-202.

2DG used herein can be administered orally or parenterally using an injection or infusion.

The dose of 2DG is not limited unless it can seriously affect patients. For example, 2DG can be administered in a dose of 100 to 5000 mg, preferably 500 to 2000 mg, per treatment.

The other antitumor agent having apoptosis-inducing activity can be administered orally or parenterally using an injection or infusion, but it is preferable to administer the agent according to the administration route approved for the agent.

It is preferable that the dose of the other antitumor agent having apoptosis-inducing activity is decided according to the dose approved for the agent, and the dose may be suitably reduced to suppress the side effect of the antitumor agent having apoptosis-inducing activity.

In addition, 2DG may be administered with glucose whose dose is preferably the equal amount of 2DG.

The suppression of the pro-survival signal by bCD is limited in only a few hours after bCD is administered. Accordingly, in case of the combination therapy with another antitumor agent, it is necessary to adjust the timing of administering the other antitumor agent to meet the time period that AKT is inactive. In case of the combination therapy with a general antitumor agent that develops its effect shortly after the administration, it is preferable to administer the antitumor agent at the same time as the administration of bCD, or about 0 to about 2 hours later. On the contrary, in case of the combination therapy with an antitumor agent that slowly develops its effect, it is preferable to administer the antitumor agent before the administration of bCD. For example, in case of the combination therapy of 2DG and bCD, 2DG takes 1 to 2 hours to develop its effect, and bCD develops its effect in 30 minutes. Thus, it is preferable to administer 2DG firstly, and then bCD 1 to 2 hours later.

Dosage forms used herein includes tablets, capsules, granules, powders, liquids, syrups, and suspensions as an oral formulation; and injections and suppositories as a parenteral formulation. These formulations can be prepared according to a conventional method. Namely, the preparations such as tablets, capsules, liquid for oral administration may be prepared by a conventional method. Tablets may be prepared by mixing the active ingredient(s) with conventional pharmaceutical carriers such as gelatin, starches, lactose, magnesium stearate, talc, gum arabic, and the like. Capsules may be prepared by mixing the active ingredient(s) with inert pharmaceutical fillers or diluents and filling hard gelatin capsules or soft capsules with the mixture. Oral liquid preparations such as syrups or elixirs are prepared by mixing the active ingredient(s) with sweetening agents (e.g. sucrose), preservatives (e.g. methylparaben, propylparaben), colorants, flavors, and the like. The preparations for parenteral administration may also be prepared by a conventional method, for example, by dissolving the active ingredient(s) of the present invention in a sterilized aqueous carrier, preferably water or a saline solution. Tablets and granules may be coated according to a well-known method. These formulations may include another ingredient having a therapeutic effect. The active ingredient (s) may be contained in 0.1 - 70 % (w/w) per the preparation.

The tumor herein means malignant tumor such as cancer, benign tumor, or neoplastic disease, which also includes hyperplasia that can be treated through the apoptosis induction of the present invention. Specific diseases of the tumor in the present invention include, but not limited thereto unless the diseases are intracerebral tumor, for example, fibrosarcoma, myosarcoma, liposarcoma, chondrosarcoma, osteogenic sarcoma, chordoma, angiosarcoma, endothelioma, lymphangiosarcoma, lymphangioendothelioma, synovioma, mesothelioma, Ewing's tumor, leiomyosarcoma, rhabdomyosarcoma, gastric cancer, esophageal cancer, rectal cancer, pancreatic cancer, ovarian cancer, prostate cancer, uterine cancer, cancer of the head and neck, skin cancer, squamous cell carcinoma, sebaceous adenocarcinoma, papillary carcinoma, papillary adenocarcinoma, cystadenocarcinoma, medullary carcinoma, bronchogenic carcinoma, renal cell carcinoma, hepatoma, Wilm's tumor, cervical cancer, testicular cancer, small cell lung carcinoma, non-small cell lung carcinoma, bladder carcinoma, epithelial carcinoma, astrocytoma, medulloblastoma, craniopharyngioma, ependymoma, pinealoma, hemangioblastoma, acoustic neuroma, oligodendroglioma, meningioma, melanoma, retinoblastoma, leukemia, lymphoma, Kaposi sarcoma, endometrial hyperplasia, focal nodular hyperplasia, prostatic hyperplasia, and primary hyperaldosteronism.

### EXAMPLE

The reagents, test methods, etc. used in the following examples are shown below.

### Reagents

As β-cyclodextrin (bCD), methyl-β-cyclodextrin (MBCD) was used for *in vitro* tests, and (2-hydroxylpropyl)-β-cyclodextrin (HPBCD) was used for *in vivo* tests.

Anti-p-PI3K antibody was obtained from Santa Cruz (sc-12929), oligoclonal anti-PI3K antibody (6HCLC) was obtained from Pierce, anti-PI3K Class II antibody (D3Q5B) was obtained from CST, anti-cytochrome c antibodies were obtained from BD Pharmingen (Cat. 556433 for blots and Cat. 556432 for microscopy), and all other primary antibodies were purchased from Cell Signaling.

Secondary antibodies conjugated with HRP were purchased from GE Healthcare and Alexa Fluor-conjugated secondary antibodies were obtained from Life Technologies.

IGF1, EGF, insulin, propidium iodide and β-cyclodextrin were purchased from Wako.

ABT-263 was purchased from Chemietek.

2-Deoxy-D-glucose, MBCD and HPBCD were purchased from Sigma.

Pan-caspase inhibitor z-VAD was purchased from Promega.

### Cell lines and cell culture

Renal cell carcinoma cell lines stably transfected with empty vector RCC4 or with vector encoding VHL were gifted from the Harada Laboratory (Kyoto University Hospital, Dept. of Anesthesia), and the cell lines UOK121 and UOK121+VHL stably transfected with VHL expression vector were gifted from Dr. Marston Linehan (Center for Cancer Research, Urologic Oncology Branch, NCI).

Panc-1 pancreatic cancer cells and A431 epidermoid carcinoma cells were also cultured in high glucose DMEM supplemented with 10% serum.

Panc-1 cells were gifted from Dr. Koji Yamada (Dept. of Bioscience and Biochemistry, Faculty of Agriculture, Kyusyu University, Japan) and A431 cells were gifted from Dr. Masaya Imoto (Dept. of Bioscience and Informatics, Faculty of Science and Technology, Keio University, Japan).

These cells and HeLa cells were all cultured in high glucose DMEM (4.5 g/ml) supplemented with 10% FBS.

The serum used herein was obtained from several different sources such as GE Health and Cosmo Bioscience.

### Western blot and immunoprecipitation

We ran 20 µg of proteins per lane for western blots. We ran 8, 10, 12.5 and 15% gels depending on the size of proteins being detected by western blots. When there are two proteins having a similar size, gel is flowed in each protein to make western blot analysis about each protein. The immunoprecipitation was undergone by adding 200 µg of protein from solubilized cells and Protein G Sepharose or Protein A Sepharose (Sigma P3296/P9242) pre-conjugated with immunoprecipitation antibody to a buffer for immunoprecipitation, and slowly rotating the tube containing the sample buffer at 4°C overnight. The buffer for immunoprecipitation comprises 20 mM Tris Ph 7.5, 1% Triton-X100, 150 mM NaCl, phosphatase inhibitor cocktail (Cell Signaling (#58709S)), and 10% glycerol. Next morning, the content in the tube was centrifuged. The precipitate was washed with the same buffer twice and dissolved in SDS buffer. The precipitate in the solution was separated with 15% SDS-PAGE or 12.5% SDS-PAGE and analyzed by Western blotting.

### FACS Analysis

After apoptosis was chemically induced, cells were washed in PBS and re-incubated in regular medium and incubated overnight. The Cell Death Assay was performed the next morning using the Propidium Iodide Incorporation assay. Cells were analyzed with BD FACS Canto II or FACS Calibur II. Results were analyzed using FlowJo. These experiments were done in triplicate, and error bars indicate the standard deviations.

### Live Cell Counts

Dead and live cells were counted by trypan-blue dye exclusion methods. These experiments were done in triplicate, and the error bars indicate the standard deviation.

### Cytochrome c release assay

RCC4 cells grown and treated on glass cover slips were first fixed with 3.7% formaldehyde in PBS for 10 minutes. Then the cover slips were briefly exposed to 100% methanol kept in a -20°C freezer. Cover slips were incubated with 10% serum in PBS before being stained with mouse anti-cytochrome c antibody overnight. Next morning, the cover slips were washed and blocked with 10% serum in PBS for 30 minutes. Then they were exposed to secondary anti-mouse antibody conjugated with AF488 for 30 minutes. They were washed again and exposed to propidium iodide (1 µg/mL in PBS) for 15 minutes to stain DNA in the nucleus before being washed and mounted on the glass slides. We used a Keyence BZ9000 microscope with a 100X objective lens for observation.

### Mouse Xenografts

Twelve-week-old NSG mice (JAX™ Mice strain NOD. Cg-*Prkdc^{scid} I12rg^{tmlWj1}*/SzJ obtained from Charles River, Japan) were engrafted with 5 X 10⁶ UOK121 cells in 0.2 ml 50% matrigel (Falcon 356234) s.c. in the lower left or right flank. Tumor-bearing mice were divided into four or five treatment groups (the first experiment and the second experiment, respectively, having at three mice to a group). They were treated orally with either 2 mg 2DG and 2 mg glucose in 0.2 ml PBS, or 2 mg HPBCD in 0.2 ml PBS. ABT-263 was initially administered orally (2 mg/kg ABT-263 in 10% ethanol, 30% polyethylene glycol 400 (Wako), and 60% Cremphore EL (Sigma). First, the 2DG/glucose mixture was administered, then two and half hours later, mice were treated with HPBCD, and thirty minutes later, mice were treated with ABT. Some mice were given all these reagents while others were given a subset of them or none at all. The first week, mice were treated twice. For the subsequent three weeks, mice were treated three times a week. Tumor size was measured three to four times a week by electronic calipers (volume = (length X width²) /2). A group of three mice were treated for each treatment condition, and the next day, tumor sizes were recorded. Error bars in the graph indicate the standard deviations. NSG mice without the UOK121 xenograft were also treated with the triple drug combination and their weights were recorded. Using blood from the tail vein, leukocyte, erythrocyte, and platelet in the blood were counted with Horiba Hematology Analyzer LC-152, and the blood glucose levels were measured with MediSafe Mini (Terumo, Japan).

### Example 1. Effect of bCD (in vitro)

Using HeLa cells which express both EGFR and IGF1R, the effects of bCD were tested about the decreases of EGF-stimulation and IGF1-stimulation to the cells. The bCD used herein was MBCD.

### (Method)

(Test A) HeLa cells were incubated in serum-free medium with 0, 1.75, 3.5, and 7.0 mM bCD for 30 minutes, which were prepared in duplicate in each bCD concentration. Among the both duplicate sets, one set of the media was stimulated with 20 ng/mL IGF1 for 20 minutes, and the other set was used as its control group. The cells were harvested and analyzed by Western Blots for phosphor-serine AKT and AKT, in which the phosphorylation of AKT was used as an indicator of the AKT activation.
(Test B and Test C) HeLa cells were incubated in serum-free medium for 30 minutes with 10 mM bCD, and a control group (untreated) was also prepared. These cells were then challenged with 100 ng/mL EGF for 0, 2.5, and 5.0 minutes in Test B, and with 10 ng/mL IGF1 for 0, 5, and 10 minutes in Test C. The cells were harvested and analyzed by Western Blots about EGFR, IGF1R, ERK, PI3K, and AKT, as well as each phosphorylation thereof. Anti PI3K Class II antibody was used for the detection of PI3K.

### (Result)

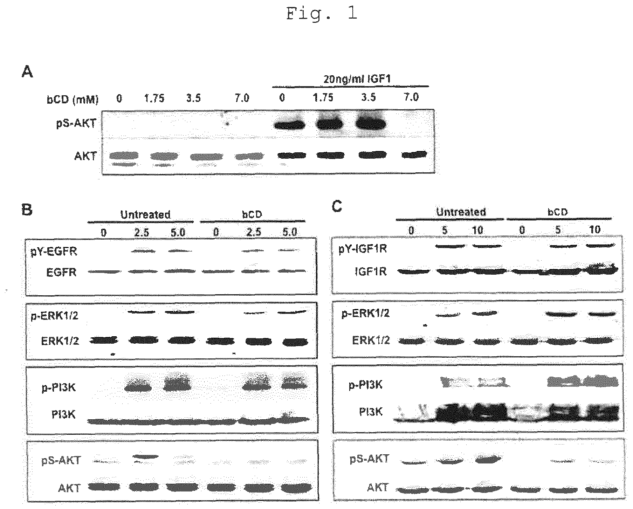

The result is shown in Fig. 1, A to C.

The results in Test A showed that 7 mM bCD was enough to completely block the process that IGF1 generates signal to AKT (Fig 1, A).

In Tests B and C, HeLa cells untreated with bCD were activated undoubtedly in both the tests. The bCD-treated cells were clearly activated in any EGFR, IGF1R, and PI3K, but AKT activations were considerably diminished (the last lanes in Fig. 1, B and C).

Thus, it is thought that bCD can interfere the signal transduction from PI3K to AKT.

### (Discussion)

- Most of RTKs activate two distinct signal transduction cascades: the RTK-Ras-ERK proliferation pathway and the RTK-PI3K-AKT pro-survival pathway.
- At the same time, the RTK-Ras-ERK signals seem to be unaffected (the second boxes in Fig 1, B and C).
- Thus, bCD disrupted the signal transduction between PI3K and AKT, and diminished PI3K-AKT pro-survival signals generated by these RTKs while leaving the Ras-ERK proliferation signals intact.

### Example 2. Synergy effect of bCD and 2DG (1)

VHL-defective renal cancer cells such as RCC4 cells are less sensitive to 2DG-ABT largely because they express IGF1R. In order to see whether 2DG-ABT combined with bCD would increase its efficacy, it should be considered that 2DG stimulates AKT phosphorylation in many cancer cell lines. Thus, we first tested whether the dual treatment of 2DG with bCD would increase or decrease AKT phosphorylation in RCC4 cells.

Since it generally takes 1-2 hours for the effect of 2DG to become noticeable, whereas bCD works within 30 minutes as shown in Example 1, RCC4 cells in serum free media are first treated with 2DG for 2 hours, and in the last 30 minutes, the cells are also treated with bCD. The bCD used herein was MBCD.

### (Method A)

RCC4 cells were incubated in serum free media for 2 hours with or without 10 mM 2DG. In the last hour, 10 mM bCD was added to each one subset of the cells treated/untreated with 2DG. Then 0-30 ng/ml IGF1 was added and the incubation continued for 5 minutes before the cells were harvested and analyzed by Western blotting.

### (Result A)

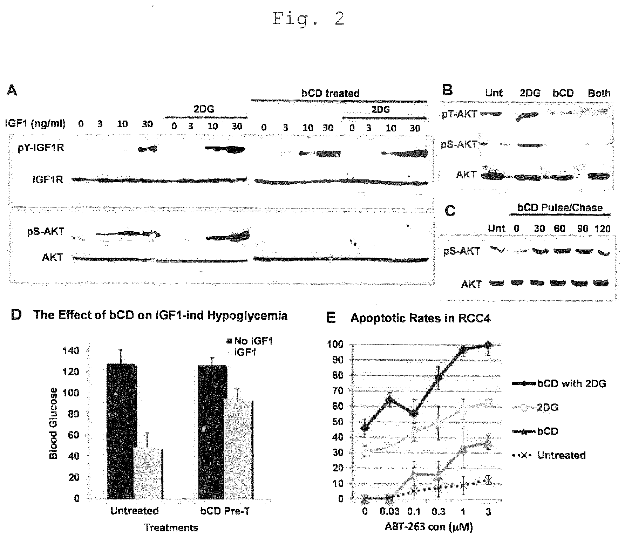

The result is shown in Fig. 2, A. the 2DG pretreatment group sensitized these cells for IGF1, increasing the phosphorylation of AKT in 2DG-treated cells. The dual treatment group with bCD, however, completely blocked AKT phosphorylation (Fig. 2, A). These results suggest that even though 2DG enhances IGF1R activities, signals from IGF1R did not reach AKT in the presence of bCD.

### Example 3. Synergy effect of bCD and 2DG (2)

In the above Example 2, the cells were incubated in serum-free medium and then stimulated with a particular growth factor, in order to test the effects of bCD on only a particular RTK. However, serum generally contains multiple growth factors as well as insulin that could activate multiple RTKs expressed in these cells. Thus, in order to test whether bCD still modulates the PI3K-AKT signals while they are continuously stimulated by serum, we treated RCC4 cells with bCD, with 2DG and with their combination, and examined the phosphorylation status of AKT. The bCD used herein was MBCD.

### (Method B)

RCC4 cells were incubated with or without 10 mM 2DG for 2 hours in the presence of 10% serum. In the last 30 minutes, each one subset of cells treated/untreated with 2DG was exposed to 10 mM bCD before the cells were harvested and analyzed.

### (Method C)

HeLa cells were incubated with 10 mM bCD for one hour in the presence of 10% serum before the cells were washed and re-incubated in medium containing 10% serum for the indicated period (∼ 120 minutes).

### (Result)

The result of Method B is shown in Fig. 2, B, and that of Method C is shown in Fig. 2, C. The phosphorylation of AKT was almost totally absent by being incubated with bCD or co-incubated with 2DG and bCD (Fig. 2, B).

However, it did not take long for the AKT activities to come back when the cells were returned to media without bCD but containing 10% serum (Fig. 2, C).

### Example 4. Effect of bCD for IGF1-induced hypoglycemia (in vivo)

Several studies had shown that when one of the bCD derivatives, hydroxypropyl-β-cyclodextrin (HPBCD) is injected into mice, the amount of bCD that remains in circulation four hours later would about 50% (J Inherit Metab Dis 2013, 36 (3) : 491-498*;* Toxicol Pathol 2008, 36 (1) :30-42). Thus, to take advantage of bCD-induced absence of pro-survival signals for cancer therapies, apoptosis needs to be induced fairly quickly. In order to see whether bCD affects PI3K-AKT pathways in animals, we did the following test. The bCD used herein was HPBCD.

### (Method D)

Five-hour starved mice were either pre-treated with 40 µg bCD for 30 minutes, or without. They were then injected with 100 ng IGF1. After thirty minutes, the blood glucose levels were measured by drawing blood from each mouse tail. The experiments were performed in triplicate samples and the error bars indicate the standard deviation. The mice were all about 20 g.

### (Result)

The result is shown in Fig. 2, D. bCD clearly attenuated IGF1-induced hypoglycemia, suggesting that bCD partially blocked the signal transduction between IGF1R and AKT in animal bodies.

### Example 5. Synergy effect of bCD and 2DG for promoting ABT-induced apoptosis

We tested if bCD in combination with 2DG-ABT can promote its apoptosis induction.

### (Method E)

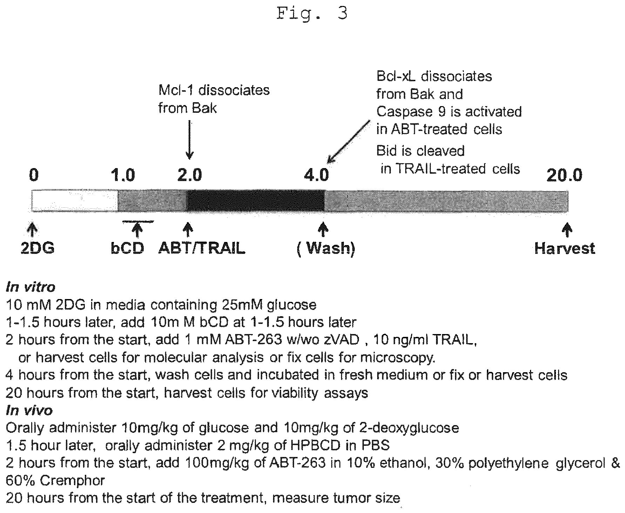

A subset of RCC4 cells was pre-incubated with 10 mM 2DG for 2 hours in the presence of 10% serum, another subset was pre-incubated with 10 mM bCD for 30 minutes in the presence of 10% serum, and yet the other subset was treated with both. And, a control group (Untreated) was also prepared. One hour after ABT addition indicated in Fig. 2E, all the cells were washed with PBS and re-incubated in the regular medium containing 10% serum overnight. The cells were harvested and analyzed for PI incorporation by FACS. The protocol is shown in Fig. 3. As bCD, MBCD was used.

### (Result)

The result is shown as a graph in Fig. 2, E.

Using 1 µM ABT-263, the 2DG-bCD-ABT combination induced apoptosis in about 95% of RCC4 cells.

Using the data from Fig. 2, E at 1 µM ABT concentration, based on the assumption that bCD and 2DG work independently, we calculated the expected outcome as 72% cell death, but the observed outcome was 97%. The chi square test of independence indicated p less than 0.0001. Thus, it was found that bCD and 2DG work synergistically for ABT-induced apoptosis.

### Example 6. Effect of 2DG-bCD-ABT triple combination across a broad spectrum of cancer cells

First, we tested the effects of bCD on AKT pro-survival signals on several cancer cell lines in Test A. Regarding A431 epidermoid carcinoma cells, we also analyzed EGF, ERK1/2, PI3K, and each phosphorylated form thereof by Western Blotting in Test B. Next, we tested the apoptosis effect in combination with ABT in Test C. The bCD used herein was MBCD.

### (Method)

(Test A) HeLa cervical cancer cells, UOK121 renal cancer cells, Panc-1 pancreatic cancer cells, and A431 squamous cancer cells were untreated, pre-treated with 10 mM 2DG, co-incubated with 10 mM bCD for the last 30 minutes, or both-treated with the 10 mM 2DG and the 10 mM bCD, in the presence of 25 mM glucose contained in the media. Each cell was harvested and analyzed by Western Blotting.
(Test B) The same samples of A431 cell lysates in Test A were analyzed for EGFR activation, ERK1/2 activation, and PI3K activation. For PI3K detection in A431 cells, anti-PI3K p85 oligoclonal antibody (6HCLC) was used.
(Test C) Untreated cells and cells treated with 2DG and bCD were prepared in the same manner as Test A. Cells incubated with 1 µM ABT for 2 hours and cells treated with all of the treatments of 2DG, bCD and ABT were also prepared. The cells were harvested and the live cells were counted by trypan-blue dye exclusion assay. Error bars indicate standard deviations from triplicate samples.

### (Result)

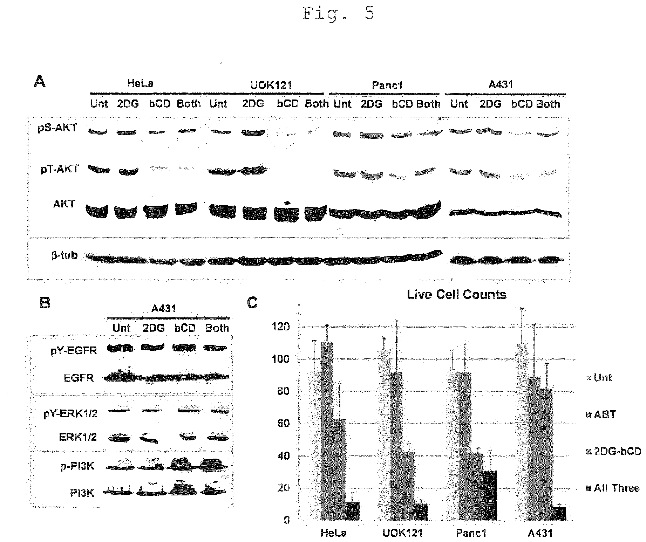

As expected, bCD attenuated AKT phosphorylation in HeLa cervical cancer cells, UOK121 renal cancer cells, Panc-1 pancreatic cancer cells, and A431 squamous cancer cells. In all, bCD attenuated AKT pro-survival signals (Fig 5, A).

Test B was performed for evaluating the effect of bCD and/or 2DG for A431 squamous cancer cells that are known to overexpress EGFR among the cancer cells used in Test A, and the results thereof showed that EGFR, ERK1/2, and PI3K were all activated (Fig. 5, B).

According to the result in Test C, the triple combination including ABT induced apoptosis in all the cell lines very effectively. Considering the results of Tests A and B, therefore it was suggested that the activity of ABT for inducing apoptosis can be enhanced by attenuating AKT pro-survival signals.

In the result of Panc-1 cells in Test C, there was not any noticeable difference between the double combination of 2DG/bCD and the triple combination including ABT, but it is thought to be because Panc-1 cells cannot take ABT intracellularly.

### Example 7. Analysis of mechanism of apoptosis induction with triple combination of 2DG-bCD-ABT

The results in the above examples show that the combination of 2DG and bCD can give a synergistic effect with overwhelming probability. In order to ensure the synergistic effect molecular-biologically, the following protocol of tests to investigate where in the cells the synergistic effect is observed was built. Firstly, proteins that precipitate with Bak were harvested and analyzed by Western Blots to identify when Mcl-1 and Bcl-xL are deleted from the Bak complex, and the microscopic examination was made to investigate when cytochrome c is released from mitochondria.

### (Method)

(A and B) RCC4 cells were treated with 2DG, bCD, 2DG + bCD, or left untreated as done in Example 6. Approximately 20 µg of the whole cell lysates (WCL) were analyzed by western blotting. Bak- and Bcl-xL-bound proteins were immune-precipitated from approximately 200 µg of cell lysates and analyzed by western blotting.
(C) 3 µM ABT with or without the 20 µM pan-caspase inhibitor z-VAD was added to the cells pre-treated with the combination of 2DG and bCD over 2 hours, and analyzed by Western blotting for caspase 9. Cleaved caspase 9 was indicated by cC9.
(D) 200 µg from the last two samples were used to precipitate Bak-bound proteins and blotted for BcL-xL and Bak.
(E) RCC4 cells treated with 2DG-bCD (left panel), RCC4 cells treated with 2DG-bCD-ABT (middle panel), and RCC4 cells treated with 2DG-bCD-ABT in the presence of 20 µM pan-caspase inhibitor z-VAD (right panel) were immunostained using anti-cytochrome c antibody and GFP-conjugated anti-mouse antibody. Nuclei were stained red with propidium iodide.

The punctate green spots appearing in cells treated with both 2DG and bCD (left panel) represent mitochondria-localized cytochrome c, while the defused stains in both middle and right panels represent cytochrome c released from mitochondria. Note: cytochrome c release takes place only after the addition of ABT. The graphic illustration of the protocol for this and other experiments is found in Fig 3.

### (Result)

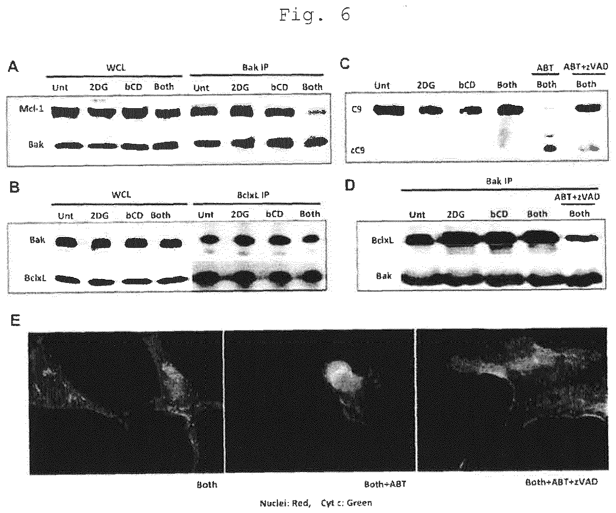

The result is shown in Fig. 6, A to E. The Mcl-1-Bak association was lost (Fig 6, A).

In contrast, ABT bound directly to Bcl-xL and caused the dissociation of the Bak-Bcl-xL complex (Fig 6, D). Only then, caspase 9 was activated (Fig 6, C).

Thus, the release of cytochrome c took place only after the addition of ABT, and it also took place in the presence of a caspase inhibitor, namely even in the absence of caspase activation (Fig. 6, D and E). Cytochrome c release was followed by caspase 9 activation.

We note that both the full release of cytochrome c and full caspase 9 activation were observed within 2 hours of ABT addition, and the apoptosis proceeded in the final step within 4 hours after first administering 2DG.

Considering these results, it has been found that both of 2DG and bCD are indispensable to sensitize cells to apoptosis caused by the release of Bak from Mcl-1 which is one of inhibitory factors of apoptosis. Namely, the synergistic effect of 2DG and bCD can release Mcl-1 from the Bak complex. In addition, it has been also found that when ABT is added, ABT binds Bcl-xL to delete Bcl-xL from the Bak complex, Bak is released from all the inhibitory factors to be activated, cytochrome c is released from mitochondria, and then apoptosis starts (see, Fig. 6, C).

### Example 8. Effect of 2DG-bCD-ABT causing tumor regression in vivo

### (Method)

(A) UOK121 cells which were human-derived cancer cells were grafted into mice, and the treatment was begun on the 7th day according to the protocol (*in vivo*) in Fig 3. Treatment groups were untreated, or treated with 2DG-ABT, HPBCD, or 2DG-HPBCD-ABT. The mice were treated further two times and tumor sizes were recorded. The error bars represent the standard deviation. Only for the mice treated with 2DG-HPBCD-ABT, the 4th treatment was carried out on 50th day.
(B) As an advanced assessment of (A), mice were divided into 5 treatment groups of untreated, or treated with 2DG-ABT, HPBCD, HPBCD-ABT, or 2DG-HPBCD-ABT, and they were treated 8 times from day 10 to day 30.

### (Result)

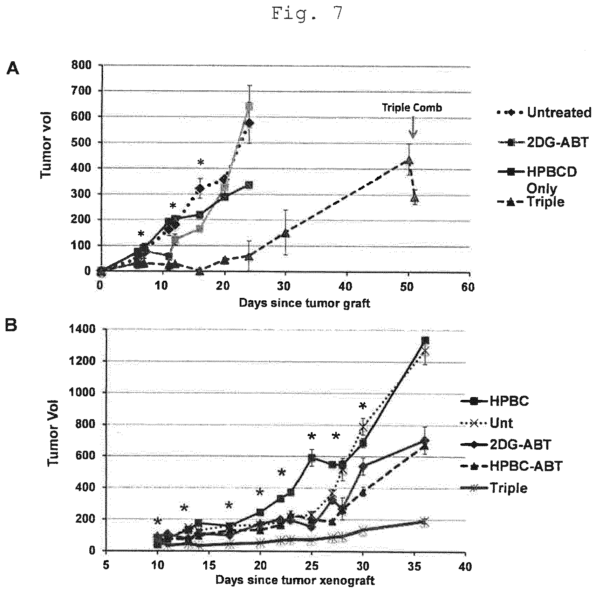

Only in the group treated with the triple combination, tumor regression was observed (Fig. 7, A). All the other mice were sacrificed because their tumors had grown to be more than 600 mm³. In the group treated with the triple combination in the first two weeks, tumors remained small (less than 60 mm³). The mice were left untreated for the subsequent weeks, during which tumors grew slowly, eventually reaching 400 mm³ on day 50.

The advanced assessment showed that only the mice treated with the triple combination responded to the treatment and tumors remained small (Fig. 7, B), which was similar to the result of Fig. 7, A. Tumors in all the mice in all the other treatment groups had grown, the tumor sized ranging from 600 - 1200 mm³ on the sixth week. In contrast, the tumors of the mice in the triple combination group grew slowly (Fig 5B).

### Example 9. Effect of 2DG-bCD-TRAIL in inducing apoptosis in pancreatic cancer cells

We hypothesized that 2DG-bCD can be combined with apoptosis inducers other than Bcl-2 antagonists, such as Fas and TNF-related apoptosis-inducing ligands (TRAIL), for efficient cancer therapy. There are reports suggesting that bCD does not interfere with death receptor activation of caspase 8, while bCD alone or together with 2DG clearly sensitizes mitochondria for induced cytochrome c release (Molecular and cellular biology 2002, 22 (1): 207-220). Thus, in type II cells, TRAIL may benefit from having mitochondria sensitized so that both the extrinsic and intrinsic pathways of apoptosis may be activated. In order to confirm the hypothesis, we did the following test.

### (Method)

(A) Panc-1 cells were treated with the standard 2DG-bCD-TRAIL protocol (Fig. 3). The live cells were distinguished from the dead cells by trypan-blue dye exclusion assays, and the live cells were counted, and graphed. The experiments were performed in triplicate and the error bars represent the standard deviations.
(B) Western blots of Panc-1 cells left untreated, treated with TRAIL, treated with 2DG-bCD combination, and treated with the combination of 2DG-bCD-TRAIL as depicted in Fig. 3. The cells were harvested at hour 6. The experiments were repeated three times with similar results.

### (Result)

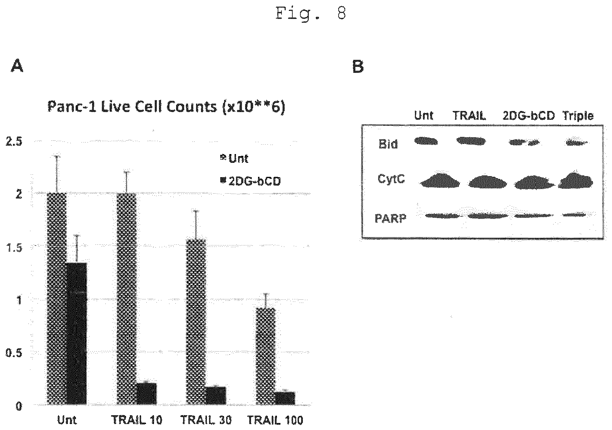

The result is shown in Fig. 8. TRAIL at 10 ng/ml concentration had virtually no effect on Panc-1 cells. However, when these cells were pre-treated with 2DG-bCD, the same 10 ng/ml TRAIL was enough to induce apoptosis in 90% of the cells. Thus, 2DG-bCD clearly sensitized Panc-1 cells for TRAIL-mediated apoptosis, suggesting that 2DG-bCD-TRAIL may be an effective treatment for pancreatic cancer.

### Example 10. Effect of different kind of cyclodextrin

We tested the effect of α-, β-, and γ-cyclodextrins on the activation of AKT and the apoptosis induction using 2DG-ABT.

### (Method)

(Test A) UOK121 cells were incubated in 10% serum medium with 5 mM or 10 mM α-, β-, and γ-cyclodextrins for 45 minutes. The same medium without any cyclodextrin was also incubated as a control. The cells were harvested and analyzed by Western Blots for the phosphorylation of AKT and ERK1/2 as an indicator of the AKT and ERK1/2 activations.
(Test B) 10 mM 2DG was added to a medium of UOK121 cells in the presence of 25 mM glucose, and the medium was incubated for 1.5 hours. To the medium was added 10 mM α-, β-, or γ-cyclodextrin, and the medium was incubated for 30 minutes. Then, 0.3 µM ABT-263 was added thereto, and the medium was further incubated for 2 hours. Separately, a control that includes only α-, β-, or γ-cyclodextrin without 2DG or ABT-263, and another control that includes neither α-, β-, or γ-cyclodextrin, nor 2DG or ABT-263 were prepared. 2.5 hours after adding each cyclodextrin (i.e., 2 hours after adding ABT-263), all the cells were washed with the medium twice, and next day the live cells were counted by trypan-blue dye exclusion assay.

### (Result)

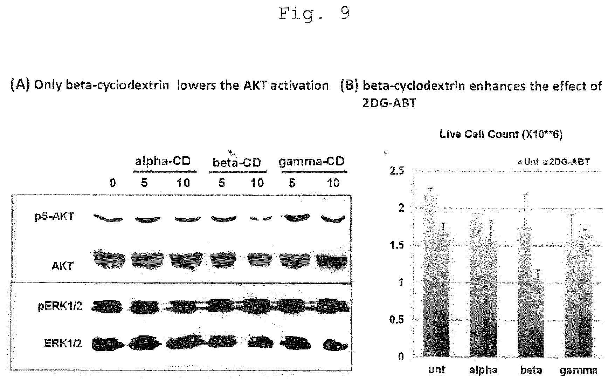

The result is shown in Fig. 9 (A) and (B). In Test A, only the AKT activation for the cells treated with β-cyclodextrin was lowered (Fig. 9, A). In Test B, the combination effect of 2DG-ABT for UOK121 cells treated with β-cyclodextrin was also clearly enhanced, but the combination effect thereof using α- or γ-cyclodextrin was little enhanced (Fig. 9B).

## Claims

1. A combination comprising β-cyclodextrin or its derivative and 2-deoxyglucose, for use in treating cancer.

2. The combination for use according to claim 1 which further comprises one or more other antitumor agents.

3. The combination for use according to claim 2 wherein the other antitumor agents comprise an antitumor agent having apoptosis-inducing activity.

4. The combination of any one of claims 1 to 3 for said use comprising administering the β-cyclodextrin or its derivative at the same time as, prior to, or after administering 2-deoxyglucose.

5. The combination of any one of claims 1 to 3 for said use comprising administering the β-cyclodextrin or its derivative 1 to 2 hours after administering 2-deoxyglucose.

6. The combination of any one of claims 2 to 5 for said use comprising administering the β-cyclodextrin or its derivative at the same time as, prior to, or after administering the other antitumor agents.

7. The combination for use of any one of claims 1 to 6 wherein the β-cyclodextrin derivative is selected from the group consisting of methyl-β-cyclodextrin (MBCD), (2-hydroxypropyl)-β-cyclodextrin (HPBCD), carboxymethyl-β-cyclodextrin, carboxymethyl-ethyl-β-cyclodextrin, diethyl-β-cyclodextrin, dimethyl-β-cyclodextrin, glucosyl-β-cyclodextrin, hydroxybutenyl-β-cyclodextrin, hydroxyethyl-β-cyclodextrin, maltosyl-β-cyclodextrin, random methyl-β-cyclodextrin, sulfobutylether-β-cyclodextrin, 2-selenium-bridged β-cyclodextrin, and 2-tellurium-bridged β-cyclodextrin.

8. The combination for use of claim 3, or of any one of claims 4 to 7 when depending on claim 3, wherein the antitumor agent having apoptosis-inducing activity is selected from the group consisting of an agent that can release Bak from Mcl-1 and/or Bcl-xL which are anti-apoptosis proteins, a Fas-related apoptosis-inducing ligand, and a TNF-related apoptosis-inducing ligand (TRAIL).

## Patentansprüche

1. Kombination, die β-Cyclodextrin oder ein Derivat davon und 2-Desoxyglucose umfasst, zur Verwendung bei der Behandlung von Krebs.

2. Kombination zur Verwendung nach Anspruch 1, die weiters ein oder mehrere andere Antitumormittel umfasst.

3. Kombination zur Verwendung nach Anspruch 2, wobei die anderen Antitumormittel ein Antitumormittel mit Apoptose-induzierender Aktivität umfassen.

4. Kombination nach einem der Ansprüche 1 bis 3 zur genannten Verwendung, die das Verabreichen des β-Cyclodextrins oder dessen Derivats gleichzeitig mit, vor oder nach dem Verabreichen der 2-Desoxyglucose umfasst.

5. Kombination nach einem der Ansprüche 1 bis 3 zur genannten Verwendung, die das Verabreichen des β-Cyclodextrins oder dessen Derivats 1 bis 2 h nach dem Verabreichen der 2-Desoxyglucose umfasst.

6. Kombination nach einem der Ansprüche 2 bis 5 zur genannten Verwendung, die das Verabreichen des β-Cyclodextrins oder dessen Derivats gleichzeitig mit, vor oder nach dem Verabreichen der anderen Antitumormittel umfasst.

7. Kombination zur Verwendung nach einem der Ansprüche 1 bis 6, wobei das β-Cyclodextrin-Derivat aus der aus Methyl-β-cyclodextrin (MBCD), (2-Hydroxypropyl)-β-cyclodextrin (HPBCD), Carboxymethyl-β-cyclodextrin, Carboxymethylethyl-β-cyclodextrin, Diethyl-β-cyclodextrin, Dimethyl-β-cyclodextrin, Glucosyl-β-cyclodextrin, Hydroxybutenyl-β-cyclodextrin, Hydroxyethyl-β-cyclodextrin, Maltosyl-β-cyclodextrin, statistischem Methyl-β-cyclodextrin, Sulfobutylether-β-cyclodextrin, 2-Selen-verbrücktem β-Cyclodextrin und 2-Tellurverbrücktem β-Cyclodextrin bestehenden Gruppe ausgewählt ist.

8. Kombination zur Verwendung nach Anspruch 3 oder einem der Ansprüche 4 bis 7 in Abhängigkeit von Anspruch 3, wobei das Antitumormittel mit Apoptose-induzierender Aktivität aus der aus einem Mittel, das Bak aus den Anti-Apoptose-Proteinen Mcl-1 und/oder Bcl-xL freisetzen kann, einem Fas-bezogenen Apoptose-induzierenden Liganden und einem TNFbezogenen Apoptose-induzierenden Liganden (TRAIL) bestehenden Gruppe ausgewählt ist.

## Revendications

1. Combinaison comprenant une β-cyclodextrine ou son dérivé et un 2-désoxyglucose, destinée à être utilisée dans le traitement d'un cancer.

2. Combinaison pour une utilisation selon la revendication 1, qui comprend en outre un ou plusieurs autres agents antitumoraux.

3. Combinaison pour une utilisation selon la revendication 2, dans laquelle les autres agents antitumoraux comprennent un agent antitumoral ayant une activité inductrice d'apoptose.

4. Combinaison selon l'une quelconque des revendications 1 à 3 pour ladite utilisation, comprenant l'administration de la β-cyclodextrine ou de son dérivé en même temps, avant, ou après l'administration de 2-désoxyglucose.

5. Combinaison selon l'une quelconque des revendications 1 à 3 pour ladite utilisation, comprenant l'administration de la β-cyclodextrine ou de son dérivé 1 à 2 heures après l'administration de 2-désoxyglucose.

6. Combinaison selon l'une quelconque des revendications 2 à 5 pour ladite utilisation, comprenant l'administration de la β-cyclodextrine ou de son dérivé en même temps, avant, ou après l'administration des autres agents antitumoraux.

7. Combinaison pour une utilisation selon l'une quelconque des revendications 1 à 6, dans laquelle le dérivé de β-cyclodextrine est choisi parmi le groupe constitué de méthyl-β-cyclodextrine (MBCD), (2-hydroxypropyl)-β-cyclodextrine (HPBCD), carboxyméthyl-β-cyclodextrine, carboxyméthyl-éthyl-β-cyclodextrine, diéthyl-β-cyclodextrine, diméthyl-β-cyclodextrine, glucosyl-β-cyclodextrine, hydroxybutényl-β-cyclodextrine, hydroxyéthyl-β-cyclodextrine, maltosyl-β-cyclodextrine, méthyl-β-cyclodextrine aléatoire, sulfobutyléther-β-cyclodextrine, β-cyclodextrine à pont 2-sélénium, et β-cyclodextrine à pont 2-tellurium.

8. Combinaison pour une utilisation selon la revendication 3, ou selon l'une quelconque des revendications 4 à 7 lorsqu'elles dépendent de la revendication 3, dans laquelle l'agent antitumoral ayant une activité inductrice d'apoptose est choisi parmi le groupe constitué d'un agent pouvant libérer Bak à partir de Mcl-1 et/ou Bcl-xL, qui sont des protéines anti-apoptose, un ligand inducteur d'apoptose lié à Fas, et un ligand inducteur d'apoptose lié à TNF (TRAIL).
